# EUROPEAN PATENT APPLICATION

(11) **EP 2 689 837 A1**
(43) Date of publication of application: **29.01.2014**
(21) Application number: 12177441.8
(22) Date of filing: 23.07.2012
(51) Int. Cl.: B01J 19/00, C07C 67/03, C07C 69/52, C11C 3/10

(54) **Process for making fatty acid lower alkyl esters via transesterification**

(71) Applicant: BASF SE, 67056 Ludwigshafen (DE)
(72) Inventor: Chan, Chee Jian, 40545 Düsseldorf (DE); Kruppa, Thomas, 47803 Krefeld (DE); Dodic, Filip, 41464 Neuss (DE)
(74) Representative: Reinhardt, Jürgen

(57) **Abstract**

Suggested is a process for making fatty acid lower alkyl esters by transesterification of fatty acid glycerides with lower aliphatic alcohols where the reaction takes place in a micro-mixer connected to a capillary, wherein
(a) fatty acid glycerides (compound a) are heated to a temperature of 45 to 65 °C;
(b) a mixture of at least one lower aliphatic alcohol and an alkaline catalyst (compound b) are preheated to a temperature of 20 to 50 °C;
(c) both compounds (a) and (b) are fed into at least one micro-mixer for transesterification, whereby the mixture is heated under a pressure of about 1.1 to about 3 bar to a temperature of about 65 to about 100 °C;
(d) the reaction mixture thus obtained and leaving the micro-mixer is fed into at least one capillary or tube for residence; and optionally
(e) the reaction product thus obtained is subjected to a separation step for purification.

## Description

### Field of invention

The present invention belongs to the area of chemical engineering and refers to a transesterification process using a micro-mixer.

### State of the art

Fatty acid alkyl esters represent important basic raw materials for a magnitude of products for all types of industries, like detergents, cosmetics, plastics, textiles etc. While in the past most of the esters were subjected to hydrogenation to produce fatty alcohols, nowadays huge amounts of these esters are used as biofuels, called "biodiesel".

Typically, fatty acid esters are obtained from renewable sources, in particular vegetable oils like palm oil, palm kernel oil, coconut oil or sunflower oil, but also from tallow or marine sources. The oils and fats represent triglycerides which are subjected to transesterification, which means that the glycerol part of the molecule is substituted by a lower alcohol, typically methanol or ethanol. Transesterification of fats and oil belong to the best established operations in industrial chemistry. The reaction can be conducted under very different conditions, for example 60 °C and 2 bar using sodium methylate as the catalyst or 220 °C and 40 bar using zinc acetate as the transesterification catalysts. In both cases it is mandatory to use the alcohol in high excess.

With respect to the enormous need for fatty acid alkyl esters, especially with respect to the booming market for biofuels, speed of reaction and selectivity are key issues. As a matter of fact, even small reductions of reaction time or minor improvements in selectivity lead to significant economic benefits. Also, reduction of the excess of alcohol is still an issue.

In recent years micro-technology has lost its image of a niche-technology, but successfully implemental into commercial processes. Also using micro-reaction systems for esterification reactions is well known from the state of the art. For example, Kulkarni et al. [Ind. Eng. Chem. Res. 46(16), p.5271-5277 (2007)] refers to the reaction of acetic acid with butanol using a combination of a micro-mixer and an isothermal micro-reactor. One of the conclusions from this article is avoiding higher reaction temperatures in order to prevent the formation of unwanted side products.

From the English abstract of Chinese patent application CN 101333451 A a method for the manufacture of biodiesel is known, involving an oil that is subjected to transesterification with a lower alcohol under alkaline conditions. The process involves a combination of a micro-mixer and a micro-reactor, however, the abstract does not mention temperature or pressure conditions.

Highly specialised micro-mixers and their use in various types of reactions are also known for example from DE 4433439 A1 (KFZ Karlsruhe) or DE 19541266 A1 (Bayer).

For example, Sun et al. [Ind. Eng. Chem. Res. 49, p 1259-1264 (2010**)]** disclose a process for transesterification of cottonseed oil with methanol including a micro-mixer connected to a delay loop. The reaction was conducted at the interface of a liquid and a gaseous phase at 60 °C and atmospheric pressure. Although the authors report that the reaction is faster compared to conventional transesterification in macroscopic reactors, under economic view there is still a need for improvement.

Therefore it has been a first object of the present invention to improve transesterification using micro-mixer systems with respect to time of reaction and selectivity. In second line the invention also addresses the issue of reducing the amount of excessive alcohol in the reaction.

### Description of the invention

Object of the present invention is a process for making fatty acid lower alkyl esters by transesterification of fatty acid glycerides with lower aliphatic alcohols where the reaction takes place in a micro-mixer connected to a capillary, wherein
(a) fatty acid glycerides (compound a) are heated to a temperature of 45 to 65 °C;
(b) a mixture of at least one lower aliphatic alcohol and an alkaline catalyst (compound b) are preheated to a temperature of 20 to 50 °C;
(c) both compounds (a) and (b) are fed into at least one micro-mixer for transesterification, whereby the mixture is heated under a pressure of about 1.1 to about 3 bar to a temperature of about 65 to about 100 °C;
(d) the reaction mixture thus obtained and leaving the micro-mixer is fed into at least one capillary or tube for residence; and optionally
(e) the reaction product thus obtained is subjected to a separation step for purification.

Surprisingly it has been found that conducting transesterification in a micro-mixer under liquid/liquid conditions, more particularly at temperatures of at least 65 °C and pressures of at least 1.1 bar, a higher mixing intensity and a faster reaction can be achieved. The invention includes the observation that under the conditions explained above less by-products are formed, thus selectivity is improved.

### Fatty acid glycerides

The nature of the fatty acid glycerides which are suitable to serve as raw materials for transesterification is not critical. Basically, they may represent vegetable oils, animal fats or even synthetic mixtures of mono-/di-/trimesters of C₆-C₂₂, preferably C₁₂-C₁₈ fatty acids and glycerol. Typical examples encompass palm oil, palm kernel oil, palm oil, sunflower oil, rapeseed oil, linseed oil, rice oil, safflor oil, olive oil, beef tallow or fish oil. The glycerides may show acid values between 1 and 50, preferably between 1 and 10, and iodine values between 0 and 120.

### Aliphatic alcohols

In the course of the transesterification glycerol is substituted by a lower aliphatic alcohol that is a C₁-C₄ aliphatic alcohol, preferably methanol or ethanol. The products obtained according to the present invention represent fatty acid C1-C4 alkyl ester, preferably methyl or ethyl esters. Preferably, the fatty acid glycerides and the aliphatic alcohols are fed into the micro-mixer in a molar ratio of about 1:2 to about 1:8. A ratio of about 1:6 is preferred, however, the invention allows also to reduce the ratio to about 1:5 t 1:5.5 without negative impact on the turn-over numbers.

### Catalyst

Although basically also heterogeneous catalyst would work, for example by coating the channels of the micro-mixer system, the preferred catalyst are homogenous. Most preferred are alcoholates which are soluble in the aliphatic alcohol, most preferred is sodium methylate, for example a 3 % w/v solution in methanol. Typically, the amount of the catalyst - calculated on the fatty acid triglycerides - ranges between about 0.5 to about 5.0, and preferably about 1 to about 2 % b.w.

### Process conditions

The essence of the present invention is to conduct the reaction within the micro-mixer in one phase under liquid/liquid conditions. This achieved by applying the pressure and temperature conditions explained above. Nevertheless, it is rather advantageous to conduct transesterification at a pressure in the range of about 2 to about 2.5, preferably about 2.1 to 2.4 bar and/or at a temperature in the range of about 70 to about 80, preferably about 75 °C.

### Micro-mixers

Micro-mixers represent reactor elements which have become well-known from the state of the art that have been published in the last years. Examples have already been mentioned in the preamble. A recent survey has been published by Hessel et al. in Chem. Engin. Sci. 60(8-9), p 2470-2501 (2005**).** As far as the nature of suitable micro-mixers needs to be illustrated, this document is hereby incorporated by reference.

The present invention encompasses embodiments according to which the reaction is conducted in more than one micro-mixer and the post-reaction takes place in more than one delay loop capillary. Preferably, at least two and at most four micro-mixers are connected either parallel or serial. Each of these micro-mixers show an average diameter of about 50 µm to about 5 mm and preferably about 100 µm to about 1 mm. Also, the process may encompass at least two and at most four delay loops connected to the micro-mixers, either parallel or serial.

### Industrial application

Due to the fact that the fatty acid lower alkyl esters according to the present invention show a significantly decreased amount of by-products, another embodiment of the present invention is related to their use as biodiesel. Biodiesel obtained according to the present invention; exhibiting for example lower amounts of hydrocarbons, shows a higher fuel value and leads to less formation of gum and carbon black in the engines.

### Example 1, Comparative Examples C1 and C2

In a first stream of sunflower oil preheated to about 60 °C and a second stream a of solution of 3 % w/v sodium methylate in methanol of about 25 °C were fed into a micro-mixer by means of two HPLC pumps. The molar ratio of methanol to oil has been 5.5:1. While passing the micro-mixer the mixtures were heated to reaction temperature. Once they left the mixer they entered a delay loop capillary for post reaction at about 60°C. Subsequently, the system was depressurised and the final product analysed by GC.
- In accordance with the invention **Example 1** was conducted at a temperature of 75 °C and a pressure of about 2.25 bar in the micro-mixer.
- **Comparative Example C1** was conducted at a temperature of 60 °C and atmospheric pressure.
- **Comparative Example C2** was conducted under standard low pressure conditions (60 °C, 2 bar) in a standard macroscopic tube bundle reactor.

Conversion of the sunflower oil versus residence time in the reaction system is reflected in Table 1.

**Table 1**

| Conversion of sunflower oil vs. residence time | | | |
|---|---|---|---|
| **Residence time** | **1** | **C1** | **C2** |
| 0 | 0 | 0 | 0 |
| 10 | 60 | 50 | 20 |
| 25 | 70 | 60 | 40 |
| 50 | 80 | 70 | 50 |
| 75 | 90 | 80 | 60 |
| 100 | 95 | 90 | 70 |
| 150 | 97 | 92 | 80 |
| 200 | 97 | 93 | 90 |
| 300 | 98 | 95 | 95 |
| 400 | 98 | 98 | 97 |
| 500 | 98 | 98 | 97 |
| 600 | 98 | 98 | 97 |

Compared to conventional low-pressure transesterification (C2) both reactions in micro-mixers are much faster and reach about 100 % conversion in about 30 % of the time. However, conducting transesterification in the micro-mixer under liquid/liquid conditions as shown in Example 1 (75 °C, 2.25 bar) instead under liquid/gaseous conditions as shown in Comparative Example C1 (60 °C, 1 bar) results in another significant increase in reaction velocity and improves the turn-over seriously.

## Claims

1. A process for making fatty acid lower alkyl esters by transesterification of fatty acid glycerides with lower aliphatic alcohols where the reaction takes place in a micro-mixer connected to a capillary, wherein
(a) fatty acid glycerides (compound a) are heated to a temperature of 45 to 65 °C;
(b) a mixture of at least one lower aliphatic alcohol and an alkaline catalyst (compound b) are preheated to a temperature of 20 to 50 °C;
(c) both compounds (a) and (b) are fed into at least one micro-mixer for transesterification, whereby the mixture is heated under a pressure of about 1.1 to about 3 bar to a temperature of about 65 to about 100 °C;
(d) the reaction mixture thus obtained and leaving the micro-mixer is fed into at least one capillary or tube for residence; and optionally
(e) the reaction product thus obtained is subjected to a separation step for purification.

2. The process of Claim 1, wherein the fatty acid glycerides represent vegetable oils, animal fats or synthetic mixtures of mono-/di-/trimesters of C₆-C₂₂ fatty acids and glycerol.

3. The process of Claim 1, wherein the fatty acid glycerides are selected from the group consisting of palm oil, palm kernel oil, palm oil, sunflower oil, rapeseed oil, linseed oil, rice oil, safflor oil, olive oil, beef tallow or fish oil.

4. The process of Claim 1, wherein the lower aliphatic alcohol is a C₁-C₄ aliphatic alcohol.

5. The process of Claim 1, wherein the lower aliphatic alcohol is methanol or ethanol.

6. The process of Claim 1, wherein the fatty acid glycerides and the aliphatic alcohols are fed into the micro-mixer in a molar ratio of about 1: 2 to about 1 : 8.

7. The process of Claim 1, wherein the catalyst is sodium methylate.

8. The process of Claim 1, wherein the amount of the catalyst ● calculated on the fatty acid triglycerides ● is about 0.5 to about 5.0 % b.w.

9. The process of Claim 1, wherein transesterification within the micro-mixer is conducted in one phase under liquid/liquid conditions

10. The process of Claim 1, wherein transesterification in the micro-mixer is conducted at a pressure in the range of about 2 to about 2.5 bar.

11. The process of Claim 1, wherein transesterification in the micro-mixer is conducted at a temperature in the range of about 70 to about 80 °C.

12. The process of Claim 1, wherein at least two and at most four micro-mixers are connected either parallel or serial.

13. The process of Claim 1, wherein the micro-mixers show average diameters of about 50 µm to about 5 mm.

14. The process of Claim 1, wherein at least two and at most four delay loops are connected to the micro-mixers, either parallel or serial.

15. Use of the fatty acid lower alkyl esters obtained according to the process of Claim 1 as biodiesel.
